# EUROPEAN PATENT APPLICATION

(11) **EP 4 371 975 A1**
(43) Date of publication of application: **22.05.2024**
(21) Application number: 22207875.0
(22) Date of filing: 16.11.2022
(51) Int. Cl.: C07C 273/16, C07C 273/18, C07C 275/62, B01J 19/00, A23K 50/15

(54) **PROCESS TO PRODUCE A MELT OF UREA AND BIURET AND SYSTEM TO PRODUCE SUCH A MELT**

(71) Applicant: Yara International ASA, 0277 Oslo (NO)
(72) Inventor: van Belzen, Ruud, 4541 HJ Sluiskil (NL); Mohan, Anand, 4541 HJ Sluiskil (NL); Lam, Robert, 4541 HJ Sluiskil (NL); Van de Walle, Tom, 4541 HJ Sluiskil (NL); Pirro, Laura, 4541 HJ Sluiskil (NL); De Vries, Pieter, 4541 HJ Sluiskil (NL)
(74) Representative: De Clercq & Partners

(57) **Abstract**

The present disclosure relates to a process and a system for producing a melt comprising urea, biuret, and a minor amount of N-containing compounds produced during a condensation process of urea, the process comprising the steps of pumping a urea melt to one or more reactor vessels each having a headspace at a top part thereof and a bottom part, subjecting the urea melt in the one or more reactor vessels to a heating process with a temperature of between 150°C to 180°C, during the heating process also subjecting the melt present in the reactor vessel(s) to a circulation process in and out of the one or more reactor vessels via a circulation loop arranged externally from each of the reactor vessels, and wherein during formation of the melt, gaseous by-products are produced which are evacuated out of the respective reactor vessel via the top part thereof, and removing the melt out of the one or more reactor vessels via the bottom part thereof.

## Description

### Technical field

The disclosure relates to a process and system to produce a melt of urea, biuret and a minor amount of N-containing compounds created during a urea condensation process.

### Background

It is generally known that, when urea is heated at temperatures starting from 130°C, it is thermally decomposed forming a condensation product, i.e., biuret. When the concentration of biuret increases and with increasing temperatures, other urea pyrolysis products or urea condensation products such as triuret, ammelide, cyanuric acid, etc. start to form. The higher the temperature, the quicker the conversion of urea to biuret.

It is now a goal of the present disclosure to provide in a simple method to produce a melt comprising urea, biuret and a relatively low number of byproducts, i.e., "minor" N-containing compounds, which are produced during the urea condensation process, without the need for additional purification steps and without the need for any additional reactants, solvents, etc.

It is a further goal of the present disclosure to provide in a method for producing such a melt which is suitable to be further processed into a non-protein nitrogen (NPN) source, more in particular serving as an animal feed-grade additive for ruminant animals and cattle.

### Summary

According to a first aspect of the present disclosure, a method is disclosed for producing a melt comprising urea, biuret, and a minor amount of N-containing compounds produced during the urea condensation process (hereafter "the melt comprising urea, biuret and N-containing compounds), the process comprising the steps of:
- pumping a urea melt to one or more reactor vessels each having a headspace at a top part thereof and a bottom part;
- subjecting the urea melt in the one or more reactor vessels to a heating process with a temperature of between 150°C and 180°C, wherein during the heating process the urea melt is converted to the melt comprising urea, biuret, and N-containing compounds,
- during the heating process subjecting the melt present in the reactor vessel(s) to a circulation process in and out of the one or more reactor vessels via a circulation loop arranged externally from each of the reactor vessels,
wherein during formation of the melt comprising urea, biuret and N-containing compounds, gaseous by-products are produced which are evacuated out of the respective reactor vessel via the top part thereof, and
- removing the melt comprising urea, biuret and N-containing compounds out of the one or more reactor vessels via the bottom part thereof.

Urea melt is liquid urea, optionally comprising a minimum amount of water. Solid urea has a melting temperature of between 130°C and 133°C, depending on the amount of water.

The advantage of the process according to the present disclosure is that it is a simple and fast production process to produce a melt as disclosed above, using no catalysts and no solvents. During this production process, only a minor amount of side products, typically less than 15 wt%, more in particular N-containing compounds such as ammelide, cyanuric acid, triuret, etc., are formed during the condensation of urea. Consequently, no further purification step is necessary before its inclusion into ruminant feed as a feed supplement. Furthermore, the circulation loop which is arranged externally from the one or more reactor vessels enhances the mixing of the melt, aids in the mass transfer and in flashing of the liquid.

In an embodiment of a process according to the present disclosure, the process is a batch process, and the residence time of the melt is between 40 minutes and 120 minutes, more in particular between 60 minutes and 90 minutes, typically depending on the quantity of melt present in the reactor vessel(s) and the process conditions in the reactor vessel(s).

In another embodiment of a process according to the present disclosure, the process is a continuous process, and the residence time of the melt in the one or more reactor vessels, is between 40 minutes and 60 minutes, typically depending on the capacity / volume of the reactor vessel and the flowrate of the urea melt.

These residence times as mentioned above are fairly short in view of the known production processes.

In a possible embodiment of a process according to the present disclosure, the process further comprises the steps of:
- introducing a carrier gas in the melt in the one or more reactor vessels which will then mix with the gaseous by-products, resulting in a gas mixture, the carrier gas more in particular being nitrogen gas, carbon dioxide, air or a mixture thereof which is bubbled in the respective reactor vessel using a sparger, and/or
- introducing a purging gas stream in the headspace of the one or more reactor vessels which will then mix with the gaseous by-products, resulting in a gas mixture, the purging gas stream more in particular being a purging air stream;
- evacuating the gas mixture out of the respective reactor vessel via the top part thereof.

The introduction of the carrier gas in the melt or the purging gas stream in the headspace of the respective reactor vessel to evacuate the formed gaseous by-products will help to accelerate the formation rate of biuret.

In an optional embodiment of a process according to the present disclosure, the gas mixture is evacuated out of the one or more reactor vessels via a pipeline to a scrubber which is arranged to purify the gaseous by-products out of the gas mixture, resulting in a purified gas. The scrubber uses more in particular water and/or acid as a scrubbing liquid.

In an additional embodiment of a process according to the present disclosure, the process further comprises the steps of
- purging part of the purified gas out of the scrubber, this part being a re-usable gas fraction,
- compressing the re-usable gas fraction to a pressure above atmospheric pressure and adding a further gas fraction with a pressure above atmospheric pressure to the re-usable gas fraction, or adding a further gas fraction to the re-usable gas fraction and compressing it to a pressure above atmospheric pressure; and
- recycling back the mixture of the further gas fraction and the re-usable gas fraction to the one or more reactor vessels, particularly as the carrier gas and/or purging gas stream.

The pressure of the re-usable gas fraction and the further gas fraction (or fresh gas) is more in particular between 1 bar and 5 bar. The pressure should be such that that there is a driving force for the gas mixture to flow from the reactor vessel to the scrubber.

In a possible embodiment of a process according to the present disclosure, the urea-containing melt in the respective reactor vessel(s) (i.e. a starting urea melt which converts to a melt comprising urea, biuret and N-containing compounds and of which the composition changes during the condensation process) is stirred using a stirrer (= agitator).

In a particular embodiment of a process according to the present disclosure, the produced melt comprises
- between 5 wt.% and 60 wt.% of urea, more in particular between 30°wt.% and 55 wt.% of urea;
- between 2 wt.% and 60 wt.% of biuret, more in particular between 30°wt.% and 50 wt.% of biuret and
- between 6 wt.% and 16 wt.% of the N-containing compounds,
in view of weight of the the total composition of the produced melt and the total composition forming 100 wt.%.

In a specific embodiment of a process according to the present disclosure, the N-containing compounds comprise
- between 3 wt.% and 10 wt.%, more in particular between 5 wt.% and 8 wt.% or between 5 wt.% and 7 wt.% of cyanuric acid,
- between 0.5 wt.% and 3 wt.%, more in particular between 0.8 wt.% and 2.0 wt.% or between 0.8 wt.% and 1.5 wt.% of ammelide, and
- between 3 wt.% and 6 wt.%, more in particular between 4 wt.% and 5.5 wt.% or between 4 wt.% and 5 wt.% of triuret;
in view of the weight of the total composition of the produced melt.

In a specific embodiment of a process according to the present disclosure, the process further comprises the steps of
- transporting the produced melt comprising urea, biuret and N-containing compounds out of the respective reactor vessel(s) to a mixing vessel, and
- adding one or more animal feed supplement compounds to the melt comprising urea, biuret and N-containing compounds in the mixing vessel and mixing it, thereby obtaining a feed supplement melt.

The one or more animal feed supplement compounds are one or more of amongst others nitrate compounds, phosphate compounds, sulphate compounds, micro-nutrients and anti-caking agents.

More in particular, the one or more feed supplement compounds are one or more nitrate compounds.

In a possible embodiment of a process according to the present disclosure, the one or more nitrate compounds are present in an amount of between 20 wt.% and 40 wt.%, in view of the total composition of the produced melt.

More in particular, the one or more nitrate compounds are chosen out of calcium nitrate, magnesium nitrate, ammonium nitrate, potassium nitrate, sodium nitrate or a mixture thereof.

In a particular embodiment of a process according to the present disclosure, the urea melt present in the one or more reactor vessels is subjected to a heating process to obtain a melt of urea, biuret and the N-containing compounds with a temperature of between 150°C and 180°C, more in particular between 160°C and 170°C, and most in particular a temperature of 165°C.

Thanks to their complex digestive system, characterized by a four-chambered stomach that allows the efficient digestion of plant materials via the microbiota installed in them, ruminants are capable of processing both true protein as well as Non-Protein-Nitrogen (NPN) from their feed. It is known that feed-grade urea, which is one source of NPN, decomposes rapidly and releases nitrogen in the form of ammonia to potentially toxic levels inside ruminant animals. Biuret provides a slower release rate of ammonia to the rumen bacteria. Biuret is less toxic compared to urea and is therefore safer to be used in ruminant feed. The production of biuret is, however, very expensive. The production process according to the present disclosure thus provides a melt which is suitable to be used more safely in animal feed in comparison with pure urea.

According to a second aspect of the present disclosure, a system for producing a melt comprising urea, biuret and a minor amount of N-containing compounds created during a urea condensation process, the system comprising
- a pumping device to pump a urea melt to one or more reactor vessels, each having a headspace located at a top part thereof, and a bottom part, wherein the one or more reactor vessels are arranged to produce the melt, and wherein each of the reactor vessels comprise
   - a heating device arranged to heat the urea melt to a temperature of between 150 °C and 180°C, wherein during the heating process the urea melt is converted in the melt comprising urea, biuret and the N-containing compounds; and
   - a circulation loop arranged externally to the reactor vessel(s) which is arranged to circulate the melt in and out of the respective reactor vessel(s); and
   - a valve arranged to evacuate gaseous by-products produced during the urea condensation process out of the respective reactor vessel(s) via the top part thereof; and
   - a valve arranged to remove the melt out of the one or more reactor vessels at the bottom part thereof.

This system is a compact system needing a minimum amount of parts to obtain the melt comprising urea, biuret and the minor amount of N-containing compounds created during the urea condensation process.

In a possible embodiment of a system according to the present disclosure, the system further comprises a valve arranged to introduce carrier gas in the melt present in the one or more reactor vessels, and a gas sparger positioned in the reactor vessel(s), wherein this carrier gas will then mix with the gaseous by-products, resulting in a gas mixture. The carrier gas can more in particular be nitrogen gas, carbon dioxide, air or a mixture thereof which is bubbled in the respective reactor vessel(s) using the gas sparger.

In another possible embodiment of a system according to the present disclosure, the system further comprises a first gas purging device arranged to introduce a purging gas stream in the headspace of the one or more reactor vessels, wherein this purging gas stream will then mix with the gaseous by-products resulting in a gas mixture. This purging gas stream is more in particular a purging air stream.

In an optional embodiment of a system according to the present disclosure, the system further comprises a scrubber which is arranged to purify the gaseous by-products out of the gas mixture, resulting in a purified gas. This scrubber more in particular uses water and/or acid as scrubbing liquid.

In a possible embodiment of a system according to the present disclosure, the system further comprises
- a second purging device to purge a part of the purified gas out of the scrubber, this part being a re-usable gas part,
- a compressor to pressurize the re-usable gas part above atmospheric pressure,
- a valve arranged to add fresh gas with a pressure above atmospheric pressure to the re-usable gas part, and
- a valve arranged to recycle back the mixture of the fresh gas and the re-usable gas part to the one or more reactor vessels.

In a particular embodiment of a system according to the present disclosure, each of the reactor vessels further comprises a stirrer, more in particular an agitator, arranged to stir the melt in the respective reactor vessel(s).

In an optional embodiment of a system according to the present disclosure, the system comprises a valve to remove the produced melt comprising urea, biuret and N-containing compounds from the one or more reactor vessels and further comprises a pipeline to transport the produced melt comprising urea, biuret and N-containing compounds from the one or more reactor vessels to a mixing vessel.

The system according to the present disclosure furthermore comprises a dosing system to add one or more nitrate compounds (in a solid or a liquid form) to the melt in the mixing vessel and a mixing device to mix the one or more nitrate compounds with the melt in the mixing vessel.

A specific embodiment of a system according to the present disclosure is arranged to perform a process according to the present disclosure as described above.

### Description of the figures

FIG. 1 shows a flow-diagram of a process to produce a melt comprising urea, biuret and a minor amount of N-containing compounds produced during the urea condensation process according to the present disclosure;
FIG. 2 shows a flow-diagram of a process to produce a melt comprising urea, biuret and a minor amount of N-containing compounds produced during the urea condensation process and one or more nitrate compounds according to the present disclosure.

### Detailed description

The present disclosure relates to the production of a melt of urea, biuret and a minor amount of N-containing compounds which are produced during the urea condensation process. The condensation of the starting urea source is obtained by applying it to a heating process. When urea is heated, starting from a temperature of around 132°C - 133°C, urea is converted into biuret and "by-products" in the form of N-containing compounds are formed. The main N-containing compounds that are formed during the condensation process of urea are ammelide, cyanuric acid and triuret. The amounts of N-containing compounds formed by the urea condensation process according to the present disclosure are only present in a "minor" amount, i.e., maximum 10 wt.% in view of the total final produced melt. As used herein, the term "urea melt" refers to the starting urea source, as further discussed below, which is provided to a reactor vessel, prior to its conversion in a heating process. As used herein, the term "melt comprising urea, biuret and N-containing compounds" generally refers to the output product stream of the reactor vessel, and corresponds to the resulting melt obtained after the condensation of the urea melt. As used herein, the term "urea-containing melt" or "melt in a reactor vessel" generally refers to the melt in a reactor vessel, subject to a heating process, with continuously changing composition due to the ongoing condensation reaction.

In the production process according to the present disclosure, a urea melt is pumped by means of a pump to one or more reactor vessels where the condensation process will take place. The urea melt provided to the one or more reactor vessels particularly comprises between 95 wt.% and 100 wt.% urea. This urea melt can originate from different sources. A first option is to directly take it out of a urea production plant with the appropriate temperature and concentration. A second option is to directly take it out of the production plant at the appropriate temperature but as a more diluted form (or solution). The concentration of a urea melt taken out of the production plant varies depending on the place where it is taken. Typical concentrations of urea originating from a urea production plant are 80 wt.%, 95 wt.% or 99 wt.% of urea. A urea concentration of 80 wt.% needs to be up concentrated by evaporating water. Urea concentrations of 95 wt.% and 99 wt.% are directly usable as the urea melt in the process according to the present disclosure. A third option is to melt solid urea.

This urea melt is then heated in the one or more reactor vessels using a heating device to a desired process temperature of between 150°C and 180°C, through which a condensation reaction of the urea occurs leading to biuret and the N-containing compounds as mentioned above. More in particular, the melt in the one or more reactor vessels is heated to a temperature between 160°C and 170°C, and most in particular a temperature of 165°C. It was surprisingly found that this process temperature range resulted in a melt comprising urea, biuret and N-containing compounds, with urea : biuret: N-containing compounds ratios which are particularly effective when the melt, following solidification, is used as a NPN feed supplement source for ruminants.

It is possible to provide a single reactor vessel or a series of different reactor vessels. Each of the reactor vessels has a top part with a headspace and a bottom part. Any water present in the melt is evaporated by the heat and is evaporated into the air present in the headspace of the respective reactor vessel(s). In these reactor vessels, the main formation of biuret and the formation of the N-containing compounds takes place, finally resulting in the desired melt according to the present disclosure.

The process according to the present disclosure can be a batch system or a continuous system. In a batch system, the formation of the melt in the one or more reactor vessels takes place during a period of between 40 and 120 minutes, more in particular between 60 and 90 minutes, depending on the quantity of melt present and the process conditions, mainly the temperature, in the reactor vessel(s). A typical residence time of the melt in the one or more reactor vessels is around 60 minutes. In a continuous system, the residence time of the melt typically takes place between 40 minutes and 60 minutes, this depending on the flowrate of the melt through the reactor vessel(s) and the volume of the reactor vessel(s).

During the heating process and the formation of the melt comprising urea, biuret and N-containing compounds, gaseous by-products are produced as well. More in particular, since urea starts to degrade, mainly ammonia (NH₃) is liberated at temperatures above the melting point of urea, which is around 132°C. Furthermore, also CO₂ (carbon dioxide) and H₂O vapor are formed. The majority thereof is produced during the initial heating of the urea. In order to increase the conversion of urea into biuret and the N-containing compounds, a first possibility is to introduce a carrier gas in the melt present in the one or more reactor vessels which will then strip the gaseous by-products out of the reactor vessel(s). The carrier gas is thus mixed with the gaseous by-products resulting in a gas mixture. A second possibility is to introduce a purging gas stream in the headspace of the one or more reactor vessels which will then mix with the gaseous by-products, resulting in a gas mixture. It is also possible to perform both of these options, either simultaneously or alternatingly.

Introducing a carrier gas in the melt present in the one or more reactor vessels is more in particular done using a sparger which is designed to bubble the carrier gas up into the melt from the bottom part of the respective reactor vessel(s). The carrier gas can be nitrogen gas, carbon dioxide, air or a mixture thereof.

Introducing a purging gas stream in the headspace of the one or more reactor vessels is more in particular done by applying a purging air stream.

The resulting gas mixtures as described above are then evacuated out of the respective reactor vessel via the top part thereof. Optionally, the produced gases are evacuated to a scrubber which is arranged to scrub the gaseous by-products out of the gas mixture, through which a purified gas is obtained. This scrubber more in particular uses water and/or acid such as sulphuric acid (H₂SO₄) or nitric acid (HNO₃) as the scrubbing liquid. The scrubber, more specifically, is an ammonia scrubber which uses acid to scrub the ammonia out of the gas mixture which is evacuated out of the top part of the respective reactor vessel.

The scrubber typically operates in a vacuum (i.e. operating at pressures less than 1 bar). The pressure in the headspace of the one or more reactor vessels should be higher than the pressure in the scrubber such that the gases should flow from the one or more reactor vessel(s) to the scrubber. The pressure in the headspace of the one or more reactor vessel(s) is typically atmospheric pressure. When a bubbling gas under a pressure above atmospheric pressure is introduced in the melt present in the reactor vessel(s), it will expand in the headspace of the respective reactor vessel(s). Between the scrubber and the one or more reactor vessel(s), a pipeline is present. Optionally, a steam ejector is arranged in this pipeline to help to lower the pressure towards the scrubber. After the scrubber, typically a ventilator is provided that is arranged to suck the gas out of the scrubber.

In order to avoid build-up of undesired gaseous by-products, such as nitrogen, the process further comprises the steps of
- purging a part of the purified gas out of the scrubber, this part being a re-usable gas part;
- compressing the re-usable gas part to a pressure above atmospheric pressure and adding fresh gas with a pressure above atmospheric pressure to the re-usable gas part, or adding fresh gas to the re-usable gas part and compressing it to a pressure above atmospheric pressure; and
- recycling back the mixture of fresh gas and the re-usable gas part to the one or more reactor vessels.

Those one or more reactor vessels are furthermore provided with a circulation loop which is arranged externally to the respective reactor vessel(s) and which is arranged to let the melt circulate through it, wherein the melt flows from the reactor vessel to the circulation loop via an inlet opening of the circulation loop and flows back to the reactor via an outlet opening of the circulation loop. This allows mixing of the melt present in the one or more reactor vessels and aids in the mass transfer. The circulation loop is optionally heated to avoid plugging of the loop which could happen when the melt crystallizes in the loop. The circulation of the melt furthermore promotes the separation of the gaseous by-products via flashing. More in particular, the outlet opening of the circulation loop, i.e. where the circulation loop is connected to the reactor vessel and where the melt flows out of the circulation loop into the respective reactor vessel, is arranged as a narrowed orifice to ensure pressure build-up and thereafter expansion of the flow of the melt, providing in amongst others a better separation of gaseous by-products and melt. Accordingly, in particular embodiments, the separation of the gaseous by-products and the urea-containing melt also comprises flashing the urea-containing melt during the circulation process, wherein the urea-containing melt is circulated back to the respective reactor vessel via an outlet opening of the circulation loop forming, said outlet opening adapted to form a constricted flow section in the circulation loop.

In order to stir the melt present in the one or more reactor vessels, a stirrer can be provided. More in particular, the stirrer is an agitator which can be used to achieve a good mixing result. The reactor vessel(s) can be a continuously stirred reactor vessel tank (CSTR). If two such CSTRs are placed in series, the size of the reactor vessels is reduced and proper mixing is ensured. More in particular, In this case, a continuously gas sparged CSTRs are used.

After the desired melt is produced, it is removed out of the one or more reactor vessels via the bottom part thereof. The produced melt can be further processed to a solid product by any known solidification technique.

The produced melt obtained by means of the above-described process according to the present disclosure more in particular comprises
- between 5 wt.% and 60 wt.% of urea, more in particular between 30°wt.% and 55 wt.% of urea;
- between 2 wt.% and 60 wt.% of biuret, more in particular between 30°wt.% and 50 wt.% of biuret and
- between 6 wt.% and 16 wt.% of the N-containing compounds,
the weight percentages being in view of the weight of the total composition of the melt comprising urea, biuret and N-containing compounds, and the total composition forming 100 wt.%.

The N-containing compounds more specifically comprise
- between 3 wt.% and 10 wt.%, more in particular between 5 wt.% and 8 wt.% of cyanuric acid,
- between 0.5 wt.% and 3 wt.%, more in particular between 0.8 wt.% and 2.0 wt.% of ammelide, and
- between 3 wt.% and 6 wt.%, more in particular between 4 wt.% and 5.5 wt.% of triuret;
the weight percentages being in view of the weight of the total composition of the produced melt comprising urea, biuret and N-containing compounds. The produced melt may also further comprise about 0.1 to 1.5 wt.% of water (based on the weight of the total composition of the melt).

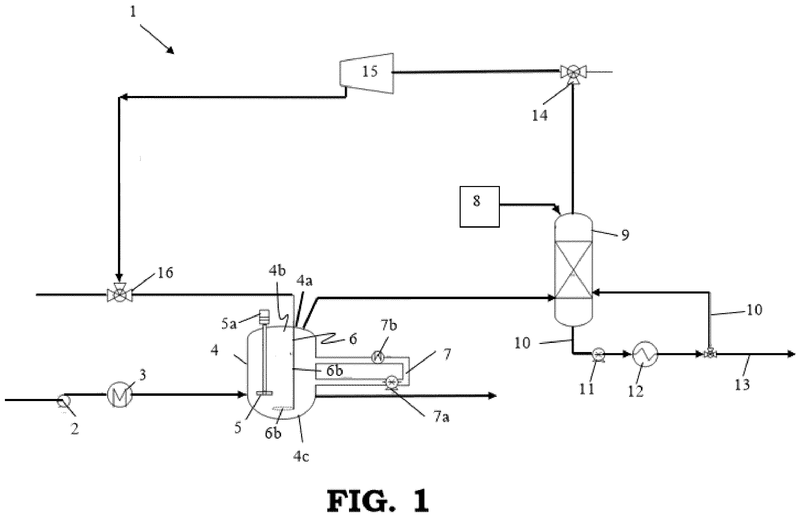

An example of a system to produce a melt comprising urea, biuret and a minor amount of N-containing compounds produced during a urea condensation process according to the present disclosure and as described above is shown in Figure 1. This system (1) comprises a pump (2) to pump a starting urea melt to a pre-heater (3) which is arranged to heat the starting urea melt to a temperature of between 130°C and 133°C, obtaining a urea melt. This urea melt is then pumped into a reactor vessel (4) in which the condensation reaction of urea melt will take place, resulting in a melt comprising urea, biuret and a minor amount of N-containing compounds. The reactor vessel (4) has a top part (4a) covering a headspace (4b) and a bottom part (4c). As already mentioned above, it is also possible to provide a number of such reactor vessels (4) in series (not shown on the figures).

The reactor vessel (4) is arranged with a heater (not shown on the figures) which is arranged to heat the melt to the desired reaction temperature of between 150°C and 180°C, in particular between 160°C and 170°C and more in particular around 165°C.

The reactor vessel (4) furthermore comprises an (eccentric) impeller or a mixer (5) which is driven by a motor or another known type of driver (5a) and which is arranged to mix the melt present in the reactor vessel (4).

The reactor vessel (4) is furthermore arranged with an externally extending circulation loop (7), comprising an inlet and outlet opening, which is arranged with a pump (7a) to pump the melt from the reactor vessel (4) in the circulation loop (7) and through the circulation loop (7). The reactor vessel (4) of this exemplary system (1) is furthermore provided with a circulation loop heater (7b) to heat the melt circulating through the circulation loop (7). The outlet of the circulation loop (7) may be adapted to restrict the flow of the melt, such as to enable the flashing of the melt.

The reactor vessel (4) is arranged with a valve (not shown on the figures) at the bottom part (4c) thereof to allow the produced melt to be drawn out of the reactor vessel (4) to be further processed.

The reactor vessel (2) is also provided with a gas sparger (6), more in particular a nitrogen (N₂) sparger, which is arranged to bubble (nitrogen) gas into the melt present in the reactor vessel (2) from the bottom to the top of the reactor vessel (4). The gas sparger (6) more in particular has a circular shaped bottom part (6a) which is furthermore perforated. The gas sparger (6) furthermore has a vertically positioned hollow pipe (6b) which is arranged to flow (nitrogen) gas through it towards the bottom part (6a). The nitrogen is sparged to strip out the ammonia (NH₃) which is formed during the production of the melt in the reactor vessel (4).

The system (1) is also arranged with a gas scrubber (10) to absorb the ammonia gas out of the gas mixture existing of ammonia gas and nitrogen gas. The scrubber (10) uses water and/or acid as scrubbing liquid (8) to selectively absorb the ammonia out of the gas mixture, producing an ammonia-water solution. The ammonia-water solution leaves the scrubber (9) at the bottom thereof. In the exemplary system as shown in Figure 1, a recycling line (10) is provided to recycle part of the ammonia-water solution back into the scrubber (9). The recycling line (10) is arranged with a pump (11) to pump the ammonia-water solution out of the bottom of the scrubber (9) back into the scrubber (9). The recycling line (10) is furthermore arranged with a heat exchanger (12) to cool the ammonia-water solution before it is recycled back to the scrubber (9) or before it is drawn out of the system (1) by means of a drainpipe (13).

The gas leaving at the top of the scrubber (9) is rich in nitrogen. To avoid build-up of inert gas in the system (1), a part of the gas leaving the scrubber (9) is purged using a valve (14). The purged nitrogen-rich gas part, which is only a small percentage of the gas coming out of the scrubber (9), is then compressed above atmospheric pressure using a compressor (15) and is mixed in a mixer (16) with fresh nitrogen. This mixture is then sent back and re-used in the reactor vessel (4).

The process according to the present disclosure may additionally comprise the step of transporting the melt out of the respective reactor vessel(s) to a mixing vessel and adding one or more feed supplement compounds, more in particular amongst others one or more nitrate compounds to the melt in the mixing vessel and mixing it, thereby obtaining a feed supplement melt. The one or more feed supplement compounds are thus more in particular added when the target percentage of biuret in the melt according to the present disclosure is obtained.

The feed supplement melt obtained via this production process according to the present disclosure more in particular comprises between 20 wt.% and 40 wt.%, and more in particular around 30 wt.%, of one or more nitrate compounds, in view of the total composition of the feed supplement melt. These one or more nitrate compounds are more in particular being chosen out of calcium nitrate, magnesium nitrate, ammonium nitrate, potassium nitrate, sodium nitrate or a mixture thereof.

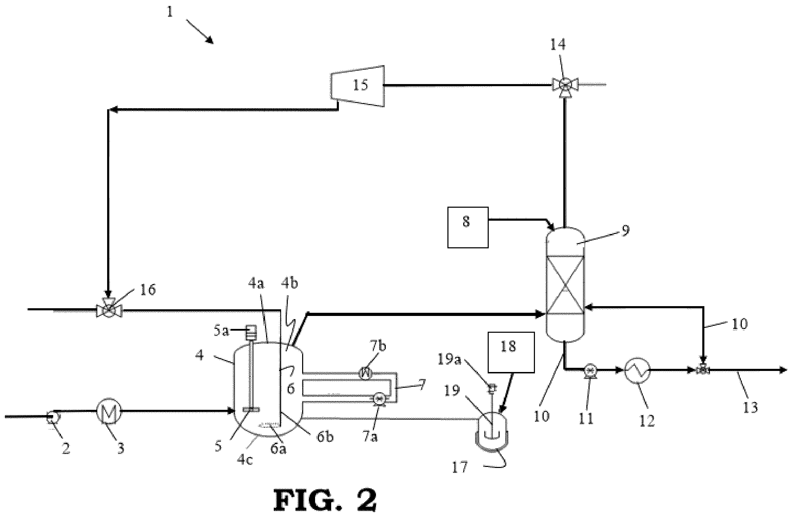

An example of a system to produce a feed supplement melt, based on a melt comprising urea; biuret, and a minor amount of N-containing compounds produced during the urea condensation process and calcium nitrate as the nitrate compound is shown in figure 2.

The production process to produce the melt according to the present disclosure comprising urea, biuret and the minor amount of N-containing compounds is the same as shown in Figure 1 and as described above. In Figure 2 however, the melt with the desired amount of urea, biuret and N-containing compounds produced during the urea condensation process is pumped to mixing vessel (17) which is arranged to add calcium nitrate (18) to the melt and to mix it using a mixer (19) which is driven by a motor (19a). The feed supplement melt is then drawn out of the mixing vessel (17) to be further processed.

## Claims

1. A process for producing a melt comprising urea, biuret, and N-containing compounds produced during a condensation process of urea, the process comprising the steps of:
- pumping a urea melt to one or more reactor vessels each having a headspace at a top part thereof and a bottom part,
- subjecting the urea melt in the one or more reactor vessels to a heating process with a temperature of between 150°C to 180°C, wherein during the heating process the urea melt is converted to the melt comprising urea, biuret, and N-containing compounds;
- during the heating process also subjecting the melt present in the reactor vessel(s) to a circulation process in and out of the one or more reactor vessels via a circulation loop arranged externally from each of the reactor vessels;
wherein during formation of the melt comprising urea, biuret, and N-containing compounds, gaseous by-products are produced which are evacuated out of the respective reactor vessel via the top part thereof; and
- removing the melt comprising urea, biuret, and N-containing compounds out of the one or more reactor vessels via the bottom part thereof.

2. A process according to claim 1, wherein, the process is a batch process, and wherein the residence time of the melt in the one or more reactor vessels is between 40 minutes and 120 minutes, more in particular between 60 minutes and 90 minutes.

3. A process according to claim 1, wherein, the process is a continuous process, and wherein the residence time of the melt in the one or more reactor vessels is between 40 minutes and 60 minutes.

4. A process according to any one of claims 1 to 3, wherein the process further comprises the steps of:
- introducing a carrier gas in the melt present in the one or more reactor vessels which will then mix with the gaseous by-products, resulting in a gas mixture, the carrier gas more in particular being nitrogen gas, carbon dioxide, air or a mixture thereof which is bubbled in the respective reactor vessel using a gas sparger, and/or
- introducing a purging gas stream in the headspace of the one or more reactor vessels which will then mix with the gaseous by-products, resulting in a gas mixture, the purging gas stream more in particular being a purging air stream; and
- evacuating the gas mixture out of the one or more reactor vessels via the top part thereof.

5. A process according to claim 4, wherein the gas mixture is evacuated out of the one or more reactor vessels via a pipeline to a scrubber which is arranged to purify the gaseous by-products out of the gas mixture, resulting in a purified gas, wherein the scrubber more in particular uses water and/or acid as a scrubbing liquid.

6. A process according to claim 5, wherein the process further comprises the steps of
- purging a part of the purified gas out of the scrubber, this part being a re-usable gas fraction;
- compressing the re-usable gas fraction to a pressure above atmospheric pressure and adding a further gas fraction with a pressure above atmospheric pressure to the re-usable gas fraction, or adding a further gas fraction to the re-usable gas fraction and compressing it to a pressure above atmospheric pressure; and
- recycling back the mixture of the further gas fraction and the re-usable gas fraction to the one or more reactor vessels as the carrier gas and/or purging gas stream.

7. A process according to any one of claims 1 to 6, wherein during the formation of the melt comprising urea, biuret, and N-containing compounds in the respective reactor vessel(s), the melt in the respective reactor vessel(s) is stirred, particularly using a stirrer.

8. A process according to any one of the preceding claims, wherein the produced melt comprising urea, biuret, and N-containing compounds comprises
- between 5 wt.% and 60 wt.% of urea,
- between 2 wt.% and 60 wt.% of biuret, and
- between 6 wt.% and 16 wt.% of the N-containing compounds,
in view of the weight of the total composition of the produced melt comprising urea, biuret, and N-containing compounds and the total composition forming 100 wt.%.

9. A process according to claim 8, wherein the N-containing compounds comprise
- between 3 wt.% and 10 wt.%, more in particular between 5 wt.% and 8 wt.% of cyanuric acid,
- between 0.5 wt.% and 3 wt.%, more in particular between 0.8 wt.% and 2.0 wt.% of ammelide, and
- between 3 wt.% and 6 wt.%, more in particular between 4 wt.% and 5.5 wt.% of triuret;
in view of the weight of the total composition of the produced melt comprising urea, biuret, and N-containing compounds.

10. A process according to any one of the preceding claims, wherein the process further comprises the steps of
- transporting the produced melt comprising urea, biuret, and N-containing compounds out of the respective reactor vessel(s) to a mixing vessel; and
- adding one or more feed supplement compounds to the produced melt comprising urea, biuret, and N-containing compounds in the mixing vessel; and
- mixing the produced melt comprising urea, biuret, and N-containing compounds and the one or more feed supplement compounds in the mixing vessel, thereby obtaining a feed supplement melt.

11. A process according to claim 10, wherein the one or more feed supplement compounds comprise one or more nitrate compounds.

12. A process according to claim 11, wherein the one or more nitrate compounds are present in an amount of between 20 wt.% and 40 wt.%, in view of the weight of the total composition of the feed supplement melt, the one or more nitrate compounds more in particular being chosen out of calcium nitrate, magnesium nitrate, ammonium nitrate, potassium nitrate, sodium nitrate or a mixture thereof.

13. A process according to any one of the preceding claims, wherein the melt present in the one or more reactor vessels is subjected to a heating process to obtain a melt of urea, biuret and the N-containing compounds with a temperature of between 150°C and 180°C, more in particular between 160°C and 170°C, and most in particular a temperature of 165°C.

14. A system for producing a melt comprising urea, biuret and N-containing compounds created during a urea condensation process, the system comprising
- a pumping device to pump a urea melt to one or more reactor vessels each having a headspace located at a top part thereof, and a bottom part, wherein the one or more reactor vessels are arranged to produce the melt comprising urea, biuret, and N-containing compounds therein, and wherein each of the reactor vessels comprise:
• a heating device arranged to heat the urea melt to a temperature of between 150°C and 180°C, wherein during the heating process the urea melt is converted in the melt comprising urea, biuret, and N-containing compounds; and
• a circulation loop arranged externally to each of the one or more reactor vessels and which is arranged to circulate the melt present in the one or more reactor vessel(s) in and out of the respective reactor vessel; and
- a valve arranged to evacuate gaseous by-products produced during the urea condensation process out of the respective reactor vessel(s) via the top part thereof; and
- a valve arranged to remove the melt out of the one or more reactor vessels at the bottom part thereof.

15. A system according to claim 14 to perform a process according to any one of claims 1 to 13.
